# EUROPEAN PATENT APPLICATION

(11) **EP 4 748 932 A1**
(43) Date of publication of application: **27.05.2026**
(21) Application number: 24383262.3
(22) Date of filing: 21.11.2024
(51) Int. Cl.: C12N 15/113, A61K 31/713

(54) **TREATMENT AND/OR PREVENTION OF ATHEROSCLEROSIS BY INHIBTION OF ANNEXIN A8**

(71) Applicant: Fundacion Instituto De Investigacion Sanitaria Fundacion Jimenez Diaz, 28040 Madrid (ES)
(72) Inventor: BLANCO COLIO, Luis Miguel, 28040 Madrid (ES); MENDEZ BARBERO, Nerea, 28040 Madrid (ES)
(74) Representative: Hoffmann Eitle

(57) **Abstract**

The present invention refers to Annexin A8 (AnxA8) inhibitors, or pharmaceutical composition comprising thereof, for use in a method for the treatment and/or prevention of atherosclerosis. Preferably, the method comprises preventing atherosclerotic plaque formation.

## Description

### FIELD OF THE INVENTION

The present invention refers to the medical field. Particularly, the present invention refers to Annexin A8 (AnxA8) inhibitors, or pharmaceutical composition comprising thereof, for use in a method for the treatment and/or prevention of atherosclerosis. Preferably, the method comprises preventing atherosclerotic plaque formation.

### STATE OF THE ART

Cardiovascular diseases (CVD) are the leading cause of death in the developed countries, accounting for nearly 18 million fatalities worldwide each year. Risk factors for atherosclerosis include high low-density lipoprotein (LDL) cholesterol levels, low high-density lipoprotein (HDL) cholesterol levels, hypertension, diabetes mellitus, family history of CVD, male gender, cigarette smoke, and high serum cholesterol levels. The main underlying cause is atherosclerosis, a multifactorial inflammatory process involving several cell types, including endothelial cells (ECs), vascular smooth muscle cells (VSMCs), and leukocytes, which interact in response to various forms of injury. Persistent hypercholesterolemia and inflammation exacerbate atherosclerotic plaque progression over time, often complicated by thrombus formation, thereby leading to the possibility of developing an acute coronary syndrome or stroke.

Annexins (Anxs) are a widespread multigene superfamily of structurally related calcium-dependent membrane-binding proteins. Anxs are involved in numerous cellular processes including vesicle trafficking, calcium signaling, cell growth or division and apoptosis. Annexin A8, like other members of the Anxs family, has been implicated in various steps of membrane trafficking. AnxA8 is located in late endosomes and is necessary for the delivery of CD63 to Weibel-Palade bodies (WPBs) and the presentation of P-selectin on the surface of human ECs, which is crucial for leukocyte adhesion.

There is an unmet medical need of finding reliable strategies aimed at treating and/or preventing atherosclerosis. The present invention is focused on solving this problem and an innovative strategy for the treatment and/or prevention of atherosclerosis is herein provided.

### DESCRIPTION OF THE INVENTION

### Brief description of the invention

The present invention refers to Annexin A8 inhibitors, or pharmaceutical composition comprising thereof, for use in a method for the treatment and/or prevention of atherosclerosis. Preferably, the method comprises preventing atherosclerotic plaque formation.

Particularly, such as it is shown in the Examples provided below, the inventors of the present invention have demonstrated that AnxA8 expression is upregulated in human and mice atherosclerotic plaques and that, more importantly, AnxA8 inhibition attenuates the progression of atherosclerosis.

So, the first embodiment of the present invention refers to Annexin A8 inhibitors, or pharmaceutical composition comprising thereof, for use in a method for the treatment and/or prevention of atherosclerosis. Alternatively, the first embodiment also refers to a method for the treatment and/or prevention of atherosclerosis in a subject, wherein the method comprises the administration of a therapeutically effective amount of an Annexin A8 inhibitor.

In a preferred embodiment, the method comprises preventing atherosclerotic plaque formation.

In a preferred embodiment, the inhibitor is a genetic inhibitor designed to interfere with the expression or function of Annexin A8 gene or protein, or a drug.

In a preferred embodiment, the inhibitor is an RNA interference molecule, CRISPR (clustered regularly interspaced short palindromic repeats) or anti-Annexin A8 antibody.

In a preferred embodiment, the inhibitor is a shRNA (short hairpin RNA), siRNA (small interfering RNA), or miRNA (microRNA).

In a preferred embodiment, the present invention refer to Annexin A8 shRNA, or pharmaceutical composition comprising thereof, for use, according to any of the previous claims, in a method for the treatment and/or prevention of atherosclerosis.

In a preferred embodiment, the shRNA is characterized by sequence SEQ ID NO: 1 (Target sequence: AGGAGTGAGATTGACTTAAAT)

The second embodiment of the present invention refers to an *in vitro* method for monitoring the efficacy of a treatment and/or for predicting the response to a treatment in a patient suffering from atherosclerosis which comprises: (a) Measuring the level of expression or activity of Annexin A8 in a biological sample isolated from the patient after the treatment; or assessing whether Annexin A8 is bound by a blocking agent (b) wherein if the level of expression or activity of Annexin A8 determined in step (a) is statistically lower than the level of Annexin A8 before the treatment, or Annexin A8 is bound by a blocking agent, it is indicative that the patient is responding to the treatment.

The third embodiment of the present invention refers to an *in vitro* method for identifying and producing candidate compounds useful in the treatment of atherosclerosis which comprises: (a) Measuring the level of expression or activity of Annexin A8 in a biological sample isolated from the patient after the administration of the candidate compound; (b) wherein if the level of expression or activity of Annexin A8 determined in step (a) is statistically lower than the level of Annexin A8 determined before the administration of the candidate compound, is indicative that the candidate compound is effective in the treatment of atherosclerosis.

In a preferred embodiment, the biological sample selected from: serum, plasma, whole blood or a biopsy of tissue (injured tissue).

For the purpose of the present invention, the following terms are defined:
- The term "comprising" includes, but it is not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.
- By "effective amount of a treatment" is intended an amount that, when administered as described herein, brings about a positive therapeutic response in the patient, for instance a relief of the symptoms. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.
- "Annexin A8 inhibitor" is any agent which binds to or interacts with human AnxA8 and inhibits the normal biological function of AnxA8 *in vitro* or *in vivo.* Non-limiting examples of categories of AnxA8 inhibitors include small molecule AnxA8 antagonists, antagonistic nucleic acid molecules (e.g., RNAi molecules) peptide-based AnxA8 antagonists (e.g., "peptibody" molecules), antibodies or antigen-binding fragments of antibodies that specifically bind human AnxA8, viral vectors that carry RNAi specific silencing construction for human AnxA8, CRISPR interference (CRISPRi) system that specifically repress the expression of human AnxA8, micro-RNA that regulate AnxA8 gene expression. Post-translational modification that altered the biological function of the gene. Moreover, drugs (e.g., Cytochalasin D, latrunculin A) that produces perturbation of the actin cytoskeleton that could affect annexin A8-mediated alterations in late endosomal appearance.

### Brief description of the figures

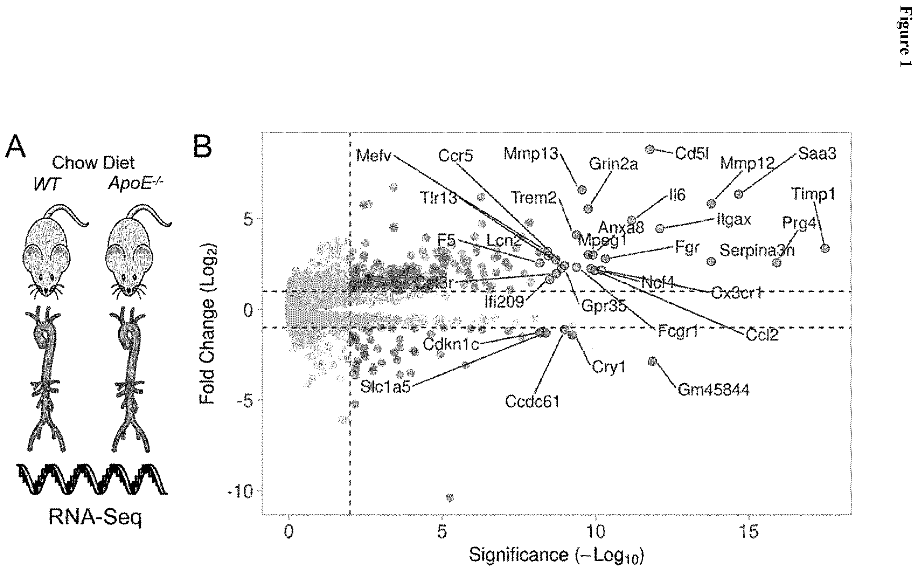

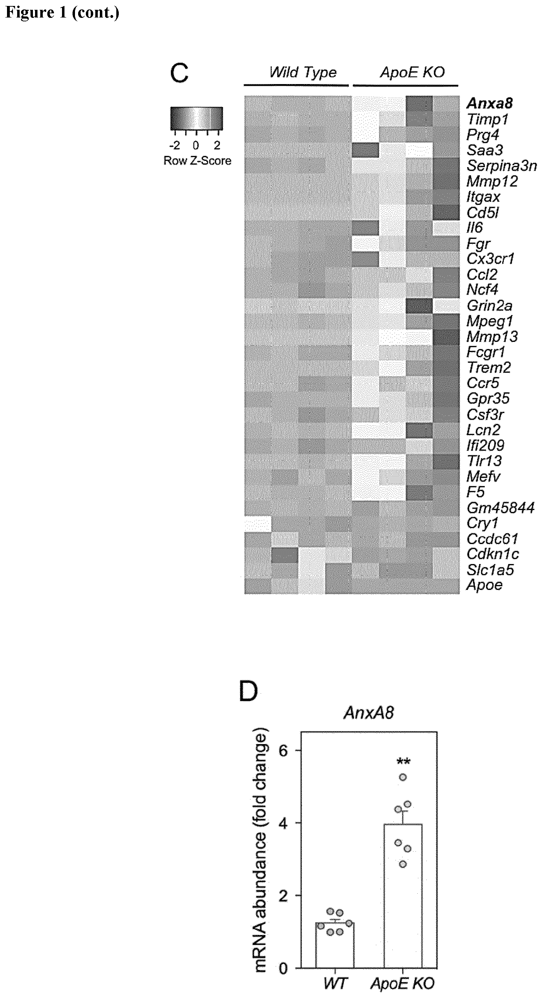

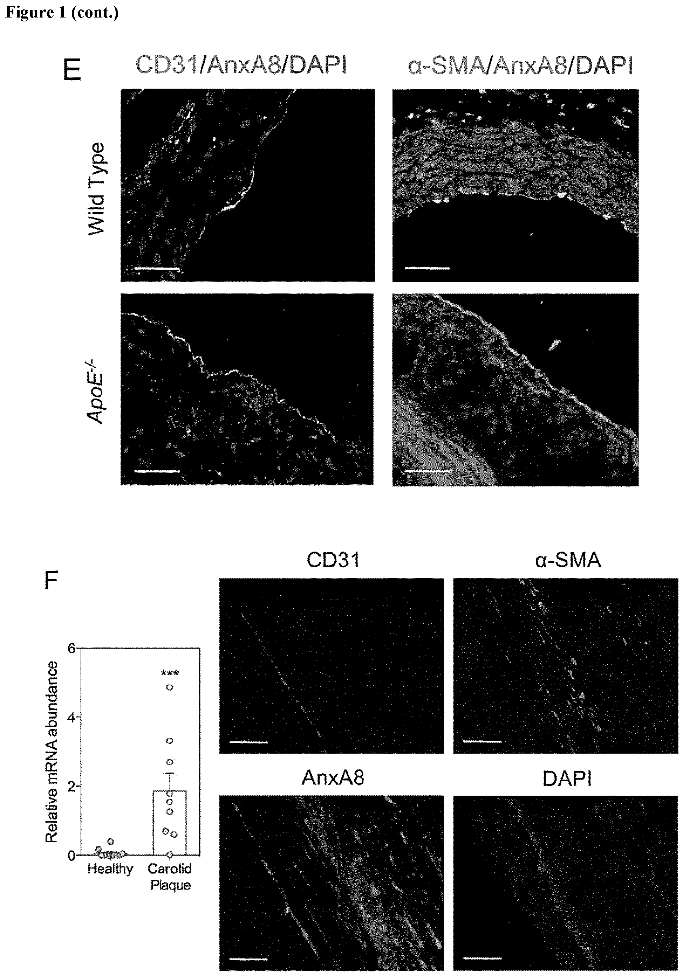

**Figure 1****. AnxA8 expression is upregulated in human and mice atherosclerotic plaques. A)** through **C),** RNA-Seq analysis of aortas from 4 *ApoE*^{*-*/*-*} mice with 4 WT mice (40-week-old male mice). **B)** Volcano plot showing the 15,643 expressed genes. Significantly differentially expressed genes are depicted in red (upregulated) or blue (downregulated) color (-adjusted *p*-value<0.01 and Log₂ fold change>1). The dashed lines indicate fold change and *p*-value significance threshold. The names indicated belong to the 30 genes with the largest statistically significant difference. **C)** Heat map representing of 30 genes with the largest statistically significant difference between wild type and *ApoE*^{*-*/*-*} mice. N=4 animals per group. **D)** Validation by RT-qPCR of *AnxA8* identified by RNA-Seq. Relative *AnxA8,* mRNA expression levels normalized to *GAPDH* mRNA expression of WT (N=6) or *ApoE*^{*-*/*-*} (N=6) aortas. Data represent mean±SEM. Student's t-test. * p<0.05 vs WT and ** p<0.01 vs WT. **E)** Immunostaining of ECs marker CD31 (red), AnxA8 (green) and their colocalization with VSMCs marker α-SMA (red) in aortic root sections of WT or *ApoE*^{*-*/*-*} mice. Scale bar, 50 µm. **F)** mRNA expression of *AnxA8* in human carotid atherosclerotic plaques (N=9) or healthy aortas (N=10) and immunohistochemistry staining of CD31, AnxA8, and α-SMA in human carotid atherosclerotic plaques. Data represent mean±SEM. Student's t-test. *** p<0.001 vs Healthy. Scale bar, 200 µm.

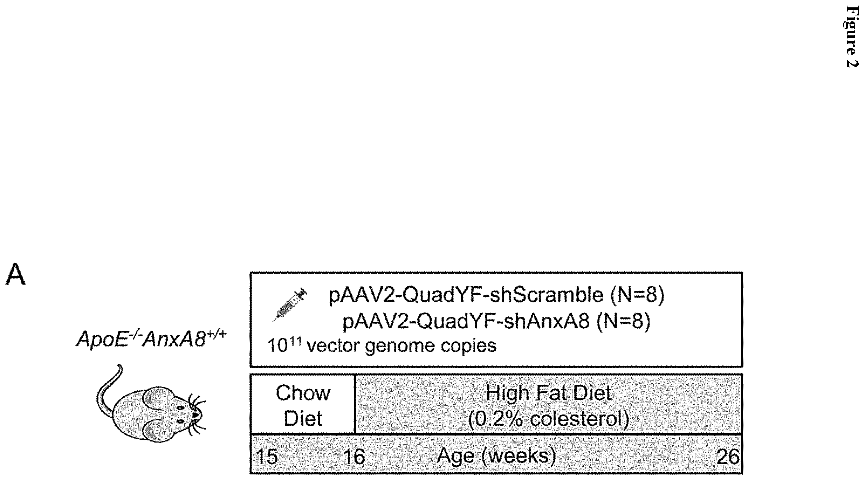

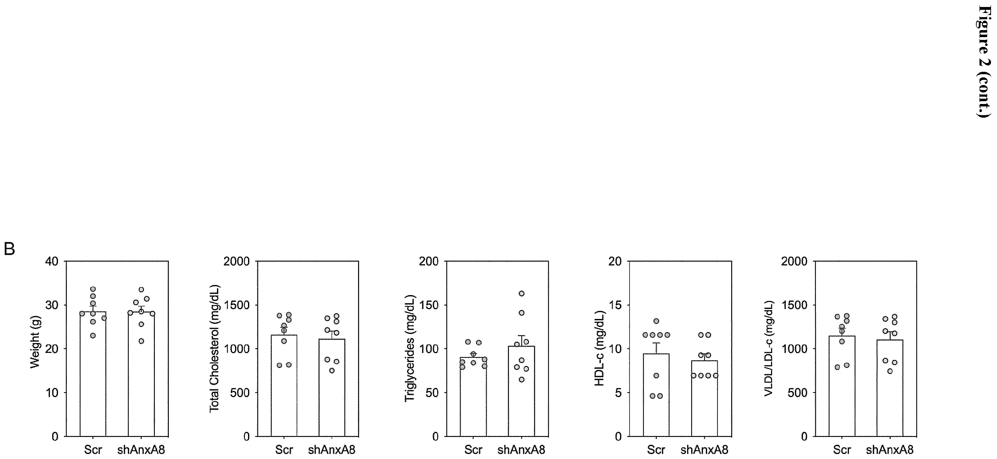

**Figure 2****. Treatment with an AnxA8 inhibitor does not modify weight or lipid levels in atheroprone mice. A)** Workflow for shAnxA8 treatment in atheroprone *ApoE*^{*-*/*-*} mice fed with a high-fat diet. To analyze the contribution of AnxA8 in the progression of atherosclerosis, 12 weeks old male *ApoE*^{*-*/*-*} mice were intravenously injected with 1×10¹¹ vp of a specific adeno-associated virus AAV2-QuadYF with tropism for endothelial cells that carry an AnxA8 short hairpain RNA (shAnxA8). AAV2-QuadYF without shRNA (Scramble) was used as control. **B)** Body weight and lipids measurement of *ApoE*^{*-*/*-*} mice after 10 weeks of treatment with pAAV2-shScramble or pAAV2-shAnxA8 (mean ± SEM; n= per group).

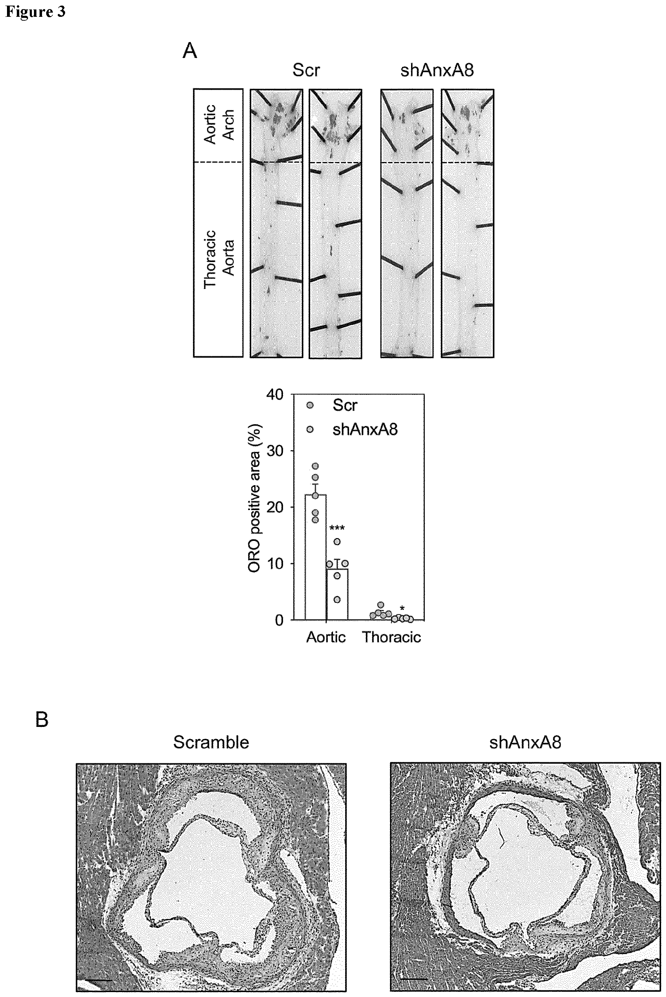

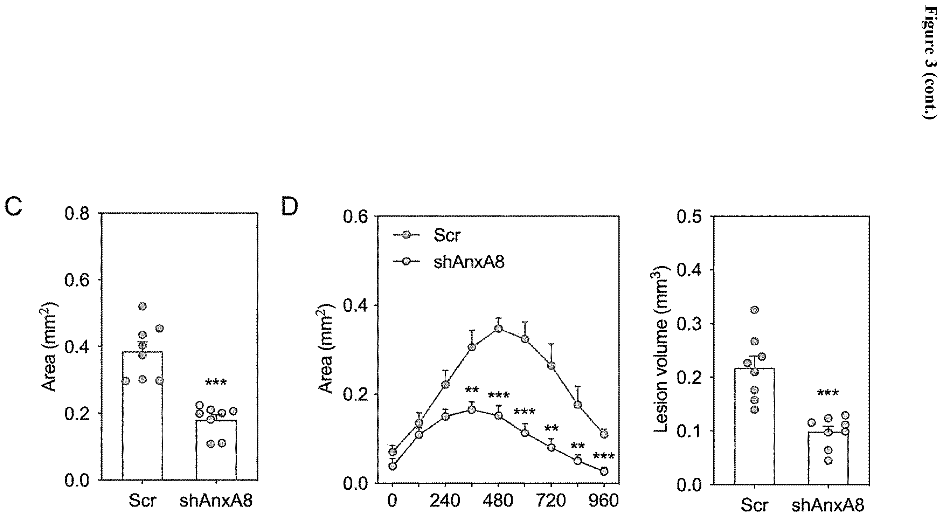

**Figure 3****. Treatment with shAnxA8 attenuates atherosclerosis progression. A)** Representative pinned-out *enface* aorta preparations and quantification of atherosclerosis in 26 weeks-old mice stained with ORO. Data represent mean±SEM of 5 animals per group. Student's t-test. * p<0.05 and *** p<0.001 vs pAAV-shScr. **B)** Representative Haematoxylin/Eosin staining and **C)** quantification of maximal lesion area in the aortic root 26 weeks-old mice. Data represent mean±SEM of 8-11 animals per group. Student's t-test. *** p<0.001 vs pAAV-shScr. Scale bars, 200 µm. **D)** Quantification of atherosclerotic lesion area along aortic root in 26 weeks-old mice. The volume of atherosclerotic lesions was estimated by calculating the area under the curve for each condition. Data represent mean±SEM of 8 animals per group. Student's t-test. ** p<0.01; ** p<0.01 *** p<0.001 vs pAAV-shScr.

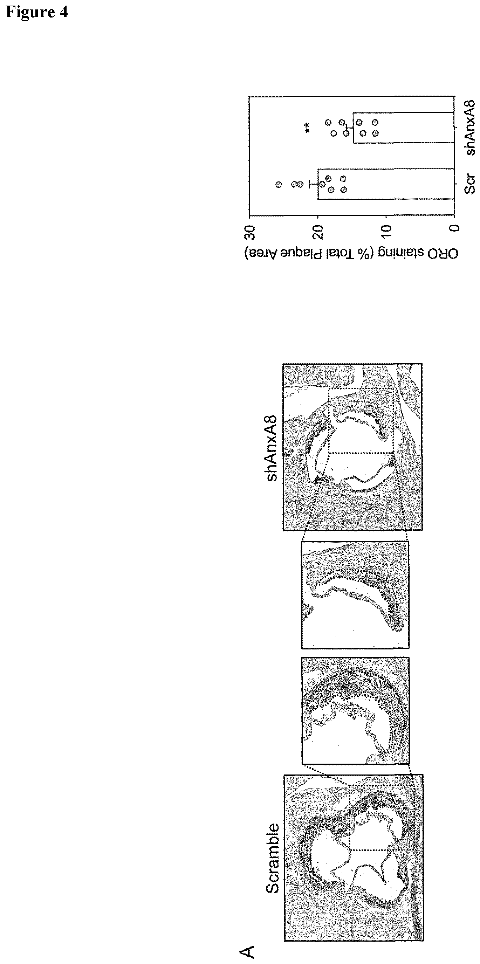

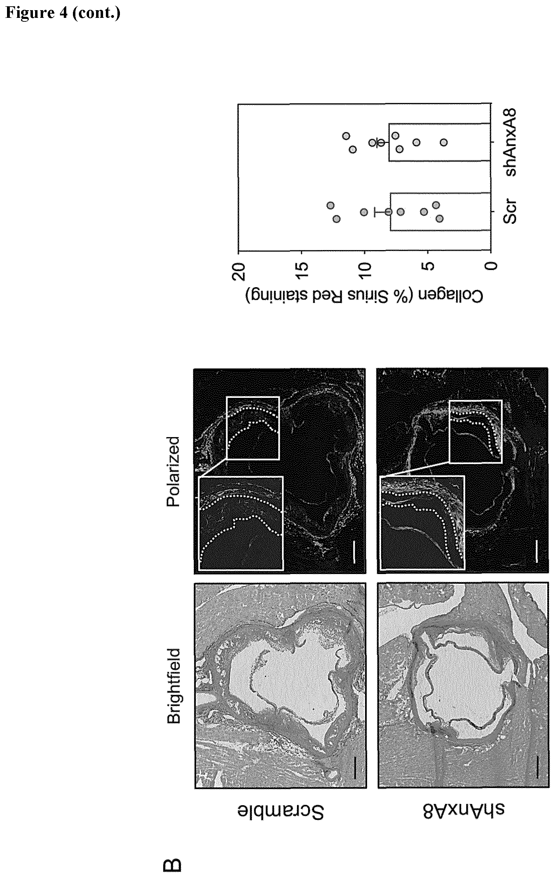

**Figure 4****. Treatment with shAnxA8 reduces lipid content in atherosclerotic plaques. A)** Representative ORO/Haematoxylin staining and quantification of ORO in the aortic root from 26 weeks-old *ApoE*^{*-*/*-*} mice. Data represent mean±SEM of 8 animals per group. Student's t-test. *** p<0.001 vs pAAV-shScr. **B)** Representative Sirius Red staining in the aortic root from 26 weeks-old *ApoE*^{*-*/*-*} mice. Quantification of the positive area is shown in the right panel. Scale bars, 200 µm. Data represent mean±SEM of 8 animals per group.

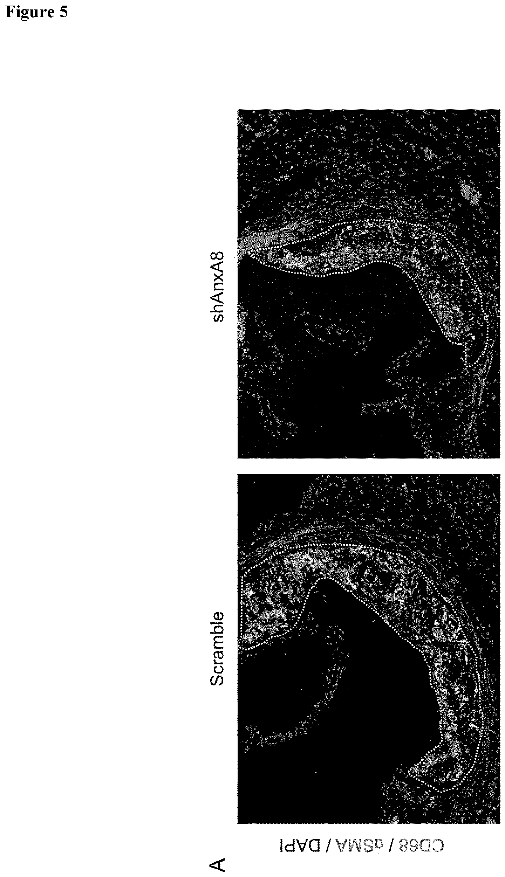

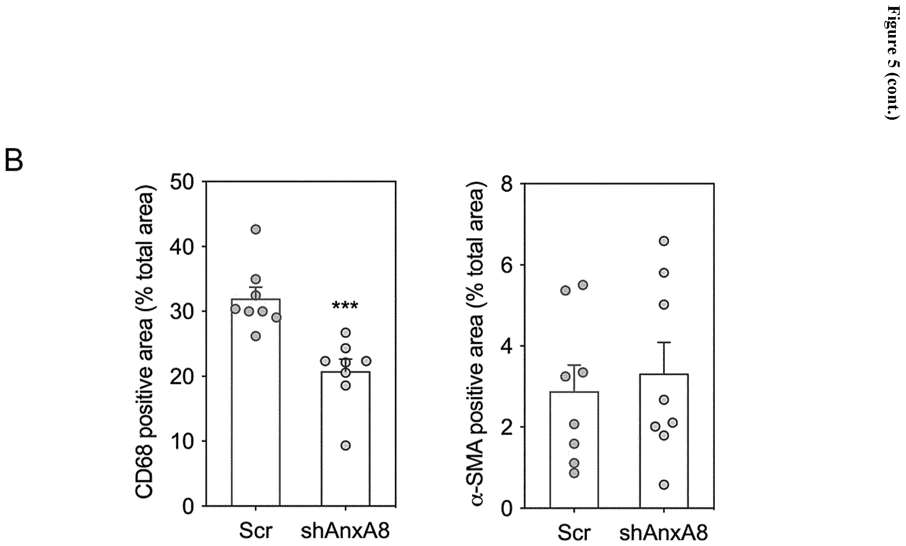

**Figure 5****. Treatment with shAnxA8 decreases macrophages content in atherosclerotic plaques. A)** Representative histological analysis of cross-sections of the aortic sinus from 26 weeks-old *ApoE*^{*-*/*-*} mice stained with CD68 (marker of macrophages), α-SMA (marker of vascular smooth muscle cells) and DAPI (nuclear staining). **B)** Quantification of macrophages or VSMCs content are shown in the panels below. Scale bars, 50 µm. Data represent mean±SEM of 8 animals per group. Student's t-test. *** p<0.001 vs pAAV-shScr.

### Detailed description of the invention

The present invention is illustrated by means of the Examples set below, without the intention of limiting its scope of protection.

### Example 1. Materials and Methods

### Example 1.1. Animals

Aorta from 40-week-old male *ApoE*^{*-*/*-*} (#002052; Jackson Laboratory) or wild type C57BL/6 mice (N=4 for both) fed on chow diet were used to obtain RNA for RNA-Seq.

To study the effect of AnxA8 inhibition on atherosclerosis progression, 15-week-old male *ApoE*^{*-*/*-*} mice were given retro-orbital injections of 1×10¹¹ vector genome copies of pAAV2-QuadYF-shScramble (N=8) or pAAV2-QuadYF-shAnxA8 (N=8). After 1 week, animals were fed on high-fat diet (HFD; 21% fat [0.2% cholesterol] +17.3% proteins; TD88137, Envigo) for 10 weeks.

All mice were maintained under barrier conditions. Water and diet were available *ad libitum.* At the end of the study, 16-hour fasted mice were anesthetized and euthanized by overdose of 100 mg/kg ketamine and 15 mg/kg xylazine and saline perfused. Blood samples were collected, and serum concentration of lipids measured by using a commercial kit adapted for a COBAS 6000 autoanalyzer (Roche Diagnostics, Rotkreuz, Switzerland). The housing and care of animals and all procedures carried out in this study were strictly in accordance with the Directive 2010/63/EU of the European Parliament and were approved by the Institutional Animal Care and Use Committee of IIS-Fundación Jiménez Diaz.

### Example 1.2. RNA-Seq library construction and sequencing

RNA-Seq libraries were prepared using the Illumina TruSeq Stranded Total RNA library prep, after ribodepletion with the Epicenter Ribozero Gold kit (cat# RZE1224) starting from 500 ng of DNAse I treated total RNA, following the manufacturer's protocol, with the exception that 14 cycles of PCR were performed to amplify the libraries, to keep the duplication rate lower than with the recommended 15 cycles. The amplified libraries were purified using AMPure beads, quantified by Qubit and QPCR, and visualized in an Agilent Bioanalyzer. The libraries were pooled equimolarly, and loaded on an Illumina HiSeq 2500 flow cell, v4 chemistry as paired end 50. The R statistical software environment was used to run the Bioconductor package, DESeq2 to analyze the RNA-Seq data set for differential expression between groups (Applied Bioinformatics Laboratory, NYU School of Medicine, New York, USA). Pairwise associations between up-regulated genes in *ApoE* KO compared to Wild type (FDR<0.05) were extracted from the STRING database with a combined score of >400. MCL clustering was performed using ClusterMaker cystoscape pluggin. Clusters with more than 4 genes were submitted to Gene Ontology (biological process) enrichment analysis implemented in Panther with mouse whole genome as background. Datasets have been deposited in NCBIGene Expression Omnibus and are accessible through GEO series number GSE207414.

### Example 1.3. En face of aorta and aortic root morphometric analysis

Atherosclerotic lesions were quantified by *en face* analysis of the whole aorta and by cross-sectional analysis of the aortic root. For *en face* preparations, the aorta was opened longitudinally, from the heart to the iliac arteries, while still attached to the heart and major branching arteries in the body. The aorta (from the heart to the iliac bifurcation) was then removed and was 'pinned out' on a white wax surface in a dissecting pan using stainless steel pins 0.2 mm in diameter. After overnight fixation with 4% paraformaldehyde and a rinse in PBS, the aortas were immersed for 1 hour in a filtered solution containing 0.2% Oil-Red O (ORO) and unstained in 80% ethanol. The aortic arch was defined as the segment between the aortic valve and the descending aorta (3-4 mm behind the left subclavian artery). The thoracic aorta was defined as the segment between the descending aorta and the renal arteries. The ORO-stained aortas were photographed and were used for quantification of atherosclerotic lesions.

Hearts containing aortic roots were carefully dissected and frozen in OCT. Aortic roots were sectioned at 5 µm thickness beginning proximally at the first evidence of the aortic valves at their attachment site of aorta. Sections were stained with ORO/hematoxylin at 100 µm intervals from 0 to the end of the aortic valves. The volume of atherosclerotic lesions was estimated by calculating the area under the curve for each condition. Maximal lesion area was calculated for each mouse by averaging the values for three sections. The necrotic core was measured as a percentage of the total plaque area and was defined as a clear area that was Masson's trichrome stain free. Boundary lines were drawn around these regions, and the area measurements were obtained by image analysis software. Fibrous cap thickness was quantified by choosing the largest necrotic core from the sections and taking a measurement from the thinnest part of the cap, determined by measuring the distance between the outer edge of the cap and the necrotic core boundary. Picrosirius red staining was performed for analysis of collagen content by measuring birefringence to plane-polarized light.

### Example 1.4. Immunohistochemical analysis

For immunohistochemical studies, patients undergoing carotid endarterectomy with asymptomatic carotid stenosis (70%) were included in the study. The region of the bifurcation of the common carotid artery was chosen. Atherosclerotic plaques were collected at the time of the surgery. Informed consent was obtained before enrolment in all cases. In addition, healthy carotid arteries were collected from deceased organ donors. Carotid samples were obtained with the authorization of the French Biomedicine Agency (PFS 09-007, BBMRI network, BB-0033-00029). The study was performed in accordance with the principles outlined in the Declaration of Helsinki, and all participants gave written informed consent. A small portion of tissue from each sample was fixed in 3.7% paraformaldehyde for immunohistochemistry assessments. In addition, atherosclerotic plaques, as well as control carotid arteries, were placed in liquid N2 for later RNA extraction.

Immunohistochemical analysis was done as previously described. Primary Abs were the monocyte/macrophage marker CD68 (Ab53444; Abcam), and the smooth muscle cell marker smooth muscle actin (Clone 1A4; Sigma), endothelial marker CD31 (ab28364; Abcam) and anti-AnxA8 (Nbp3-05654; NobusBio). For human samples, anti-AnxA8 (AF8105; R&D Systems) was used. Incubation without primary Abs and/or irrelevant species- and isotype-matched immunoglobulins was used as a negative control for all immunostainings. Anti-rat Alexa Fluor 488 and anti-rabbit Alexa Fluor 488 or 543 were used as secondary Ab. Computer-assisted morphometric analysis was performed with Image-Pro Plus software (version 1.0 for Windows). The threshold setting for area measurement was equal for all images. Samples from each animal were examined in a blinded manner. Results were expressed as % positive area vs total atherosclerotic area.

### Example 1.5. RT and real-time PCR analysis

Human atherosclerotic tissues, aortic wall tissues from healthy human controls (0.2 g for both), and total aorta from *ApoE*^{*-*/*-*} or wild type mice were snap-frozen in liquid nitrogen, homogenates were resuspended in TRIzol buffer (Life Technologies), and total RNA was purified. Duplicate samples were quantified by determining absorbance at 260 nm. Real-time PCR was performed on a TaqMan ABI 7700 Sequence Detection System using heat-activated TaqDNA polymerase (Amplitaq Gold). After an initial hold of 2 minutes at 50°C and 10 minutes at 95 °C, the samples were cycled 40 times at 95 °C for 15 seconds and 60 °C for 60 seconds. The expression of target genes was normalized to housekeeping transcript [18S/glyceraldehyde-3-phosphate dehydrogenase *(GADPH)].*

TaqMan probes Human *AnxA8* (Hs04190981) was purchased from Applied Biosystems and optimized according to the manufacturer's protocol. Mouse mRNA levels for *Anxa8,* was done by amplification of cDNA using SYBR Premix Ex TaqTM (Takara Biotechnology). All measurements were performed in triplicate. The amount of target mRNA in samples was estimated by the 2ΔCT relative quantification method. Values of each sample were obtained as multiples of their baseline values.

### Example 1.6. Statistical Analysis

Data are presented as mean ± the standard error of the mean (SEM) and the statistical significance of the differences was evaluated with the Student's *t* test or ANOVA Tukey's post-hoc for multiple comparations. Significance was accepted at the level of p<0.05. Data analysis was performed using GraphPad Prism 6.0a software (GraphPad, San Diego, CA).

### Example 2. Results

### Example 2.1. AnxA8 expression is upregulated in human and mice atherosclerotic plaques

To unveil genes that are differentially expressed in atherosclerosis, mRNA from aortas of 40-week-old atherosclerosis-prone *ApoE*^{*-*/*-*} mice (N=4) were compared with aortas of sex- and age-matched wild-type (WT) mice (N=4) fed with chow diet to avoid spontaneous plaque formation. Reproducibility was high at the level of individual samples as evident from principal component analysis (PCA). RNA-Seq analysis revealed 743 genes differentially expressed in *ApoE*^{*-*/*-*} mice, 483 up- and 260 downregulated (adjusted *p* value by FDR<0.05). As expected, main functions enriched in up-regulated genes in *ApoE*^{*-*/*-*} clustered according to their functional associations are related to inflammatory and immune response processes. Among the differentially regulated transcripts, we identified many genes that have been previously associated with atherosclerotic plaque development, including chemokines such as *Ccl2* and *Cx3cl1,* interleukins such as *IL-6,* metalloproteinases or their inhibitors such as *Mmp12, Mmp13* and *Timp1,* or the proto-oncogene *Fgr* (**Figure 1A-C**)**.** Additionally, we also found several up- or down-regulated genes whose role during atherosclerosis progression has not been previously characterized. To confirm the results obtained in RNA-Seq, *AnxA8* mRNA expression was independently validated by qRT-PCR in new set of aortas isolated from both *ApoE*^{*-*/*-*} and WT mice. In line with the data obtained in RNA-Seq, *AnxA8* was significantly up-regulated in aortas of *ApoE*^{*-*/*-*} mice compared with those from WT mice (**Figure 1D**)**,** with *AnxA8* being one of the most significantly up-regulated genes in both RNA-Seq and qRT-PCR analysis (**Figure 1A-D**)**.**

Immunohistochemistry analysis of aortic sinus showed that AnxA8 colocalized with the EC marker CD31 in WT mice (**Figure 1E**)**.** AnxA8 was expressed mainly in ECs in atherosclerotic plaques from *ApoE*^{*-*/*-*} mice (**Figure 1E**)**.** In line with the results obtained in the murine model, human carotid atherosclerotic plaques also showed significantly higher *AnxA8* mRNA expression compared with healthy arteries (**Figure 1F**)**,** and AnxA8 co-localized markedly with CD31 and α-SMA in human carotid plaques (**Figure 1F**)**.**

### Example 2.2. AnxA8 inhibition attenuates the progression of atherosclerosis

To analyze the contribution of AnxA8 in the progression of atherosclerosis, 15-week-old atheroprone *ApoE*^{*-*/*-*} mice were given retro-orbital injections of 1×10¹¹ vector genome copies of pAAV2-QuadYF-shScramble (N=8) or pAAV2-QuadYF-shAnxA8 (N=8). After 1 week, animals were fed on high-fat diet for 10 weeks. No differences were observed in body weight or serum cholesterol, triglycerides, HDL-c or LDL-c concentrations between the different groups (**Figure 2**). We assessed the atherosclerotic lesions in the entire aorta by *en face* staining with Oil-Red O to visualize lipid-laden atherosclerotic plaques. We found a 60% reduction of the *en face* aortic arch lesion area in *ApoE*^{*-*/*-*} mice treated with shAnxA8 compared to shScr treatment (**Figure 3A**)**.** In addition, a marked reduction was observed in the atherosclerotic lesion size and volume at the aortic root *ApoE*^{*-*/*-*} mice treated with shAnxA8 compared to shScr treatment (≈55% reduction for both; **Figure 3B-D****).**

The composition of the lesion, rather than size of the plaque, determines the likelihood of its rupture. Whereas collagen fibers stabilize atherosclerotic plaques, lipid depositions make plaques more prone to rupture. We observed a marked reduction in lipid deposition in the aortic wall of *ApoE*^{*-*/*-*} mice treated with shAnxA8 compared to shScr treatment (**Figure 4A**)**.** Collagen content was observed between groups of treatment were similar between both groups of mice **(****Figure 4B**)**.**

Macrophage foam cell formation as a result of the excess of lipid deposition is a critical step in atherosclerotic plaque development. Analysis of atheroma revealed decreased accumulation of CD68⁺ cells, without affecting smooth muscle cell content in *ApoE*^{*-*/}*⁻AnxA8*^{*-*/*-*} mice treated with shAnxA8 compared to shScr treatment **(****Figure 5**)**.**

Collectively, these results demonstrate that AnxA8 inhibition attenuates plaque progression and promotes plaque stability in atheroprone mice. The findings of our study suggest that interventions capable of reducing AnxA8 expression may delay atherosclerotic plaque progression.

## Claims

1. Annexin A8 inhibitor, or pharmaceutical composition comprising thereof, for use in a method for the treatment and/or prevention of atherosclerosis.

2. Annexin A8 inhibitor, or pharmaceutical composition comprising thereof, for use, according to claim 1, wherein the method comprises preventing atherosclerotic plaque formation.

3. Annexin A8 inhibitor, or pharmaceutical composition comprising thereof, for use, according to any of the previous claims, wherein the inhibitor is a genetic inhibitor designed to interfere with the expression or function of Annexin A8 gene or protein, or a drug.

4. Annexin A8 inhibitor, or pharmaceutical composition comprising thereof, for use, according to any of the previous claims, wherein the inhibitor is an RNA interference molecule, CRISPR or anti-Annexin A8 antibody.

5. Annexin A8 inhibitor, or pharmaceutical composition comprising thereof, for use, according to any of the previous claims, wherein the inhibitor is a shRNA, siRNA, or miRNA.

6. Annexin A8 shRNA, or pharmaceutical composition comprising thereof, for use, according to any of the previous claims, in a method for the treatment and/or prevention of atherosclerosis.

7. Annexin A8 shRNA, or pharmaceutical composition comprising thereof, for use, according to any of the previous claims, wherein the shRNA is **characterized by** sequence SEQ ID NO: 1.

8. *In vitro* method for monitoring the efficacy of a treatment and/or for predicting the response to a treatment in a patient suffering from atherosclerosis which comprises: (a) Measuring the level of expression or activity of Annexin A8 in a biological sample isolated from the patient after the treatment; or assessing whether Annexin A8 is bound by a blocking agent (b) wherein if the level of expression or activity of Annexin A8 determined in step (a) is statistically lower than the level of Annexin A8 before the treatment, or Annexin A8 is bound by a blocking agent, it is indicative that the patient is responding to the treatment.

9. *In vitro* method for identifying and producing candidate compounds useful in the treatment of atherosclerosis which comprises: (a) Measuring the level of expression or activity of Annexin A8 in a biological sample isolated from the patient after the administration of the candidate compound; (b) wherein if the level of expression or activity of Annexin A8 determined in step (a) is statistically lower than the level of Annexin A8 determined before the administration of the candidate compound, is indicative that the candidate compound is effective in the treatment of atherosclerosis.

10. *In vitro* method, according to any of the claims 8 or 9, wherein the biological sample selected from: serum, plasma, whole blood or biopsy of tissue.
